# EUROPÄISCHE PATENTSCHRIFT

(11) **EP 0 994 696 B1**
(45) Veröffentlichungstag und Bekanntmachung des Hinweises auf die Patenterteilung: **18.02.2004**
(21) Anmeldenummer: 98933605.2
(22) Anmeldetag: 12.06.1998
(51) Int. Cl.: A61K 9/50, A61K 31/401

(54) **KONTROLLIERT FREISETZENDE PHARMAZEUTISCHE ZUBEREITUNG MIT EINEM ACE-HEMMER ALS WIRKSTOFF**
CONTROLLED RELEASE PHARMACEUTICAL PREPARATION WITH ACE INHIBITOR AS ACTIVE AGENT
PREPARATIONS PHARMACEUTIQUES A LIBERATION CONTROLEE CONTENANT UN INHIBITEUR DE L'ENZYME DE CONVERSION DE L'ANGIOTENSINE COMME PRINCIPE ACTIF

(30) Priorität: 12.06.1997 DE 19724696
(43) Veröffentlichungstag der Anmeldung: 26.04.2000
(73) Patentinhaber: HEXAL AG, D-83607 Holzkirchen (DE)
(72) Erfinder: FISCHER, Wilfried, D-83607 Holzkirchen (DE); KLOKKERS, Karin, D-83607 Holzkirchen (DE); OPPELT, Renate, D-83607 Holzkirchen (DE)
(74) Vertreter: Boeters, Hans Dietrich, Dr.
(86) Internationale Anmeldenummer: PCT/EP1998/003536
(87) Internationale Veröffentlichungsnummer: WO 1998/056355

(56) Entgegenhaltungen:
- WO-A-96/29994
- DE-A- 4 431 832
- US-A- 5 158 777

## Beschreibung

Die vorliegende Erfindung betrifft eine pharmazeutische Zubereitung, mit der sich eine verbesserte Wirkstofffreisetzung in Abhängigkeit von Zeit und vom pH-Wert der Umgebung erreichen läßt. Insbesondere betrifft die Erfindung eine derartige Zubereitung mit einem ACE-Hemmer (angiotensin converting enzyme) als Wirkstoff, vorzugsweise mit Captopril.

Retardarzneiformen für die kontrollierte und verzögerte Freisetzung von Captopril sind bekannt. So beschreibt US-A-5 158 777 eine Zusammensetzung, bei der ein Teil des Wirkstoffs (Captopril) sofort und ein zweiter Teil verzögert freigesetzt wird. Dies wird gemäß Beispiel 2 dadurch erreicht, daß man zwei verschiedene Pelletarten vorsieht, von denen die eine wirkstoffhaltige Pelletart unbeschichtet ist, während die andere wirkstoffhaltige Art einen Kern aufweist, der u. a. Captopril und Ascorbinsäure enthält, wobei der Kern mit einem Methacrylsäure-Polymeren (Eudragit RS) überzogen ist, das eine verzögerte Wirkstofffreisetzung bewirkt.

Weitere Retardformen von Captopril umfassen beispielsweise gemäß US-A-4 666 705 eine unbeschichtete Tablette mit einem Gehalt an einem Acrylsäurepolymeren, gemäß US-A-5 738 850 eine Zubereitung mit einem Gehalt an Captopril in Kombination mit Chitosan und gemäß US-A-4 756 911 eine beschichtete Tablette, die einen Kern mit beispielsweise Captopril als Wirkstoff (Spalte 4 Zeile 57), ein oder mehrere wasserlösliche oder wasserquellbare primäre hydrokolloide Quellmittel mit einem Methoxylgehalt, ein oder mehrere sekundäre hydrokolloide Quellmittel, ein oder mehrere nicht quellbare Bindungsmittel und/oder Wachse und ein oder mehrere Schmiermittel umfaßt.

Aus DE 4.431832 sind pharmazeutische Zubereitungen bekannt, die Captopril als Initialdosis und weiteres Captopril enthalten, wobei die Zubereitungen mit mindestens zwei verschiedenen Beschichtungen versehen sind.

Es hat sich jedoch gezeigt, daß der Stand der Technik dahingehend verbesserungsbedürftig ist, daß der Wirkstoff nach dem Stand der Technik vorzeitig freigesetzt wird und so zu einem zu kurz anhaltenden therapeutischen Plasmaspiegel führt. Auch können bei einigen Retardformen die Beschichtungen noch nicht genügend aufgelöst sein, um den Wirkstoff freizusetzen, wenn die Tablette den Darm erreicht hat, so daß der Wirkstoff vor der Resorption aus dem Magen/Darmtrakt ausgeschieden wird, da im Dickdarm keine Resorption mehr erfolgt.

Bei der Erfindung zugrundeliegenden Untersuchungen hat sich gezeigt, daß Formulierungen nach dem Stand der Technik zwar in vitro retardierende Effekte zeigen, sich in vivo jedoch keine über einen längeren Zeitraum konstante und therapeutisch wirksame Blutspiegelkonzentration bzw. länger anhaltende ACE-Inhibierung erreichen läßt.

Aufgabe der Erfindung ist es, eine pharmazeutische Zubereitung, insbesondere eine Zubereitung mit einem ACE-Hemmer als Wirkstoff vorzusehen, beispielsweise mit Captopril, die insbesondere bei einer Einmaldosis eine kontrollierte Wirkstofffreisetzung erlaubt und so über einen längeren Zeitraum einen therapeutisch wirksamen Blutspiegel mit möglichst geringen Schwankungen der Blutspiegelkonzentration gewährleistet und dem Bedürfnis der so-fort einsetzenden Wirkung nachkommt und ferner eine langanhaltende ACE-Hemmung ermöglicht.

Dazu wird erfindungsgemäß eine pharmazeutische Zubereitung vorgesehen, die folgende Komponenten umfaßt oder aus ihnen besteht:
(i) eine Wirkstoff-Initialdosis, die durch den Wirkstoff gegebenenfalls in Form von Pulver, Granulat und/oder Pellets vorgesehen wird, jeweils neben fakultativen Hilfsstoffen,
(ii) eine erste verzögert freisetzende Art Pellets, bei denen der Wirkstoff sowie fakultative Hilfsstoffe von einer Beschichtung umhüllt sind, und
(iii) eine zweite verzögert freisetzende Art Pellets, bei denen der Wirkstoff und fakultative Hilfsstoffe wiederum von einer Beschichtung umhüllt sind,
   - wobei der Wirkstoff ein ACE-Hemmer ist und
   - wobei die Massen der Beschichtungen gemäß (ii) und (iii) auf Gewichtsbasis im Verhältnisbereich von 1 : 2 bis 1 : 7 vorliegen.

Mit der erfindungsgemäßen Zubereitung, konnte man für Captopril als Wirkstoff feststellen, daß sich in vivo Blutspiegelkonzentrationen mit äußerst geringen Schwankungen einstellen lassen und zudem eine nahezu sofortige Wirkung des Medikaments einsetzt. Überraschend war, daß der Wirkstoff aus der erfindungsgemäßen Zubereitung so freigesetzt wird, daß ausgeprägte Blutspiegelspitzen zu Beginn vermieden und dennoch therapeutisch wirksame Blutkonzentrationen über lange Zeit erhalten werden. Vor allem wurde überraschenderweise gefunden, daß eine überdurchschnittlich anhaltende ACE-Hemmung erzielbar ist.

Bei der erfindungsgemäßen Zubereitung können die Massen der Beschichtungen gemäß (ii) und (iii) auf Gewichtsbasis im Verhältnis von etwa 1 : 5 vorliegen.

Bei dem Wirkstoff handelt es sich also um einen ACE-Hemmer (angiotensin converting enzyme), insbesondere um Captopril, Moexipril, Perindopril, Quinapril, Ramipril, Spirapril, Tandolapril, deren Gemische und/oder pharmazeutisch unbedenkliche Salze, beispielsweise Hydrochloride, beispielsweise Perindoprilerbumin.

Bei der Initialdosis kann der Wirkstoffgehalt 5 bsi 30 Gew.-% des Gesamtwirkstoffgehaltes betragen.

In der Initialdosis kann der Wirkstoff als Pulver, Granulat und/oder als Pellet vorliegen, wobei Granulat und Pellets übliche Hilfsstoffe enthalten können.

Pellets der ersten und Pellets der zweiten verzögert freisetzenden Art können dadurch erhalten werden, daß Pellets, die gegebenenfalls für eine Initialdosis hergestellt wurden, mit der jeweiligen Beschichtung versehen werden.

Bei der Beschichtung der ersten und der zweiten verzögert freisetzenden Pelletart kann es sich um eine magensaftresistente Beschichtung handeln, vorzugsweise auf Basis von Polymethacrylsäure, insbesondere auf Basis von Eudragit S. Bei einer bevorzugten Ausführungsform wird für die erste und die zweite verzögert freisetzende Pelletart dasselbe Beschichtungsmaterial gewählt.

Die Beschichtung der ersten und der zweiten verzögert freisetzenden Pelletart weist bei einer bevorzugten Ausführungsform außer Polymethacrylsäure keine weitere Komponente gleicher oder größerer Acidität auf.

Die Beschichtung der ersten und/oder zweiten verzögert freisetzenden Pelletart kann übliche Filmbildner und/oder Hilfsstoffe umfassen, vorzugsweise Dibutylphthalat, Polyethylenglycol, Triethylcitrat (Citroflex), Ethylcellulose (Aqua coat), Titandioxid und/oder Hydroxypropylmethylcellulose. Als Hilfsstoffe können mikrokristalline Cellulose und/oder Lactose in Betracht kommen.

Das Gewichtsverhältnis von Initialdosis zu erster verzögert freisetzender Pelletart zu zweiter verzögert freisetzender Pelletart kann im Bereich von 1 : 1 : 1 bis 1 : 10 : 10 liegen und insbesondere etwa 1 : etwa 1 : etwa 2 betragen.

Die erfindungsgemäße Zubereitung kann durch folgende Gewichtsverhältnisse gekennzeichnet sein:
10 bis 30 Gew.-% Initialdosis,
20 bis 40 Gew.-% erste verzögert freisetzende Pelletart und
40 bis 60 Gew.-% zweite verzögert freisetzende Pelletart,
wobei alle drei Komponenten insgesamt 100 Gew.-% ausmachen; sie kann insbesondere gekennzeichnet sein durch
etwa 22,9 Gew.-% Initialdosis,
etwa 25,8 Gew.-% erste verzögert freisetzende Pelletart und
etwa 51,3 Gew.-% zweite verzögert freisetzende Pelletart,
wobei alle drei Komponenten insgesamt 100 Gew.-% ausmachen.

Die erfindungsgemäße Zubereitung kann in Form einer Kapsel, insbesondere einer Gelatinekapsel vorliegen, wobei die Kapsel alle drei Komponenten umfaßt. Eine derartige Kapsel kann eine für eine Tagesdosis oder für eine Einmaldosis erforderliche Wirkstoffmenge umfassen. So kann eine Kapsel eine für die Tagesdosis oder Einmaldosis erforderliche Captoprilmenge umfassen, insbesondere im Bereich von 25 bis 300 mg, vorzugsweise 50 bis 200 mg und speziell 75 bis 150 mg.

Dabei kann auf Basis einer Tagesdosis oder Einmaldosis die Captopril-Initialdosis 5 bis 30 mg betragen.

Die Pellets können 10- bis 50-proz. im Wirkstoffgehalt sein, wobei zur Pelletierung übliche Hilfsstoffe, wie mikrokristalline Die Pellets können 10- bis 50-proz. im Wirkstoffgehalt sein, wobei zur Pelletierung übliche Hilfsstoffe, wie mikrokristalline Cellulose und/oder Lactose, verwendet werden können und wobei die Pellets der drei Komponenten unterschiedlich im Wirkstoffgehalt sein können.

Das Verhältnis der Wirkstoffgehalte von Initialdosis : erste verzögert freisetzende Pellets: zweite verzögert freisetzende Pallets kann zwischen 1 : 3 : 3 und 1 : 10 : 10 oder zwischen 1 : 2,5 : 4 und 1 : 10 : 10 liegen und insbesondere etwa 1 : 2,5 : 4 betragen. Es kann somit auch zwischen 1 : etwa 2,5 : 3 und 1 : 10 : 10 liegen.

Nachstehend wird die Erfindung anhand von Beispielen und Figuren näher erläutert. Es zeigen
**Figur 1** den retardierenden Effekt einer Captopril-Kapsel gemäß Beispiel 1 im Vergleich zu einer schnell freisetzenden Captopril-Tablette;
**Figur 2** einen Captopril-Plasmaspiegel im Bezug zur ACE-Inhibierung entsprechend zu Figur 1;

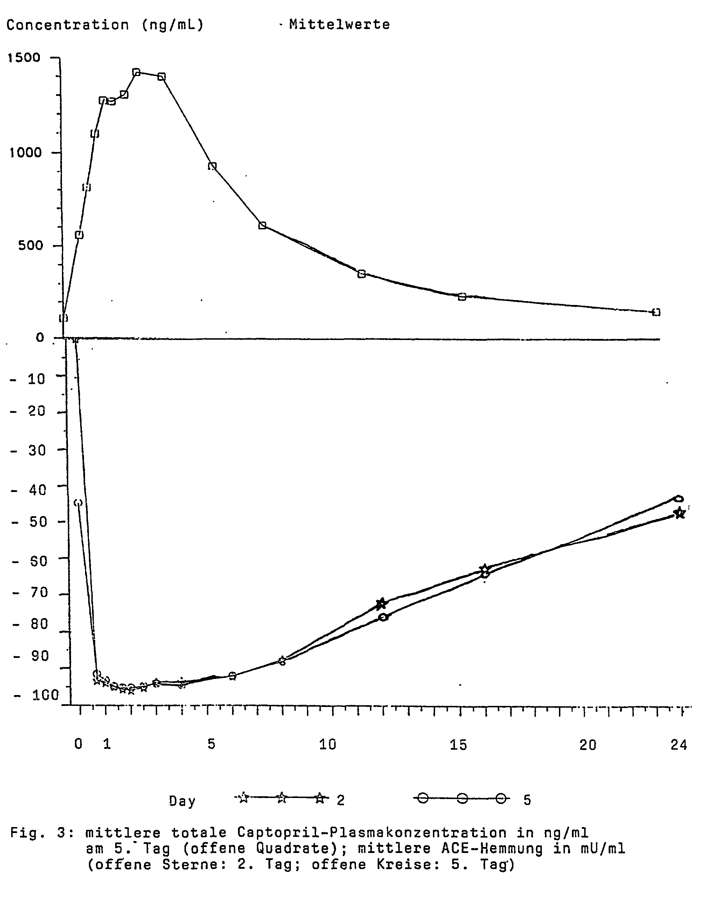

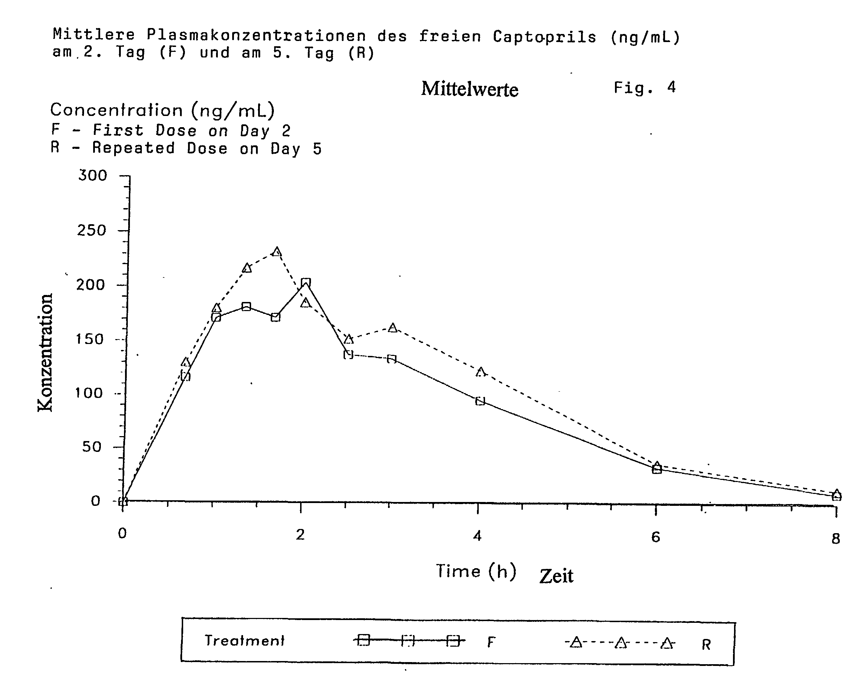

**Figur 3** einen Captopril-Plasmaspiegel in Bezug zur ACE-Hemmung entsprechend zu Beispiel 2 und Figur 4 und
**Figur 4** die mittlere Plasmakonzentration des freien Captoprils entsprechend zu Figur 3.

### Beispiel 1

### A) Herstellung

Für Captopril-Retardkapseln wurden die folgenden drei Pelletarten vorgesehen.

Pellet 1; die Zusammensetzung war wie folgt:

| | |
|---|---|
| Captopril | 5 mg |
| Avicel (mikrokristalline Zellulose) | 3 mg |
| Tablettose | 2 mg |

Pellet 2; 700 g Pellet der Art 1 wurden zunächst mit 40,48 g OPADRY II und 250 g Wasser lackiert. Die Lösung für eine zweite Lackschicht setzte sich wie folgt zusammen:

| | |
|---|---|
| Eüdragit S 100 | 62.5g |
| Dibutylphthalat | 6,25 g |
| Ethanol 96 % | 350,00 g |
| gereinigtes Wasser | 87,5 g |

Pellet 3; 700 g Pellet der Art 1 wurden mit 40,48 g OPADRY II und 250 g Wasser vorlackiert. Die Lösung für eine zweite Lackschicht setzte sich wie folgt zusammen:

| | |
|---|---|
| Eudragit S 100 | 192,5 g |
| Dibutylphthalat | 19,25 g |
| Ethanol 96 % | 1078 g |
| gereinigtes Wasser | 269,5 g |

Zur Herstellung von Captopril-Retardkapseln wurden 100 mg Pellets der Art 1, 700 mg Pellets der Art 2 und 700 mg Pellets der Art 3 in eine Gelatinekapsel gefüllt. Damit ergab sich eine Wirkstoffgesamtkonzentration an Captopril von 150 mg.

### B) Pharmacokinetische und pharmacodynamische Untersuchungen

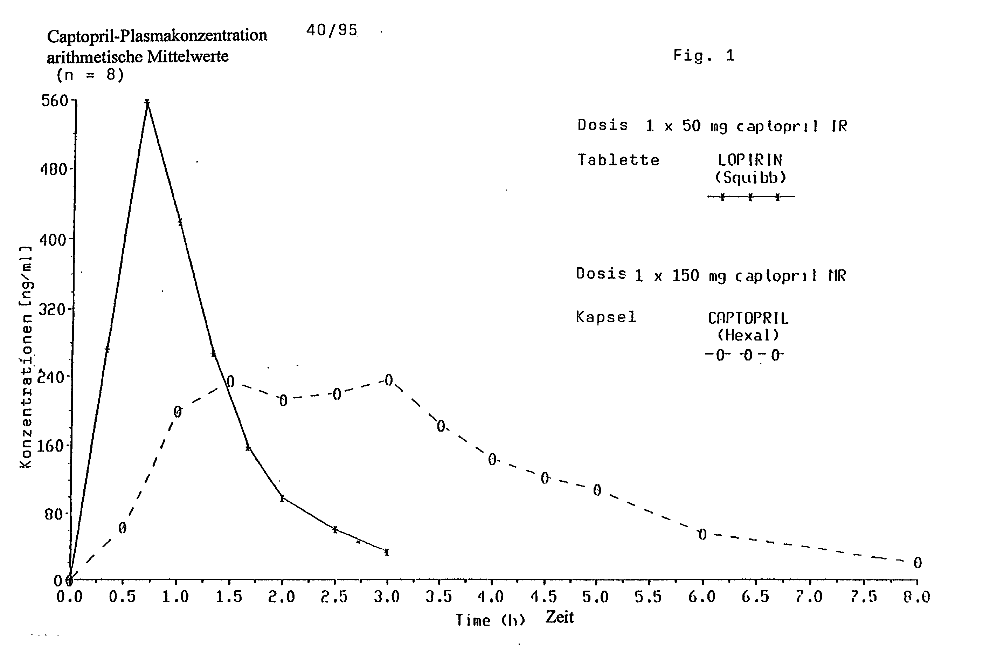

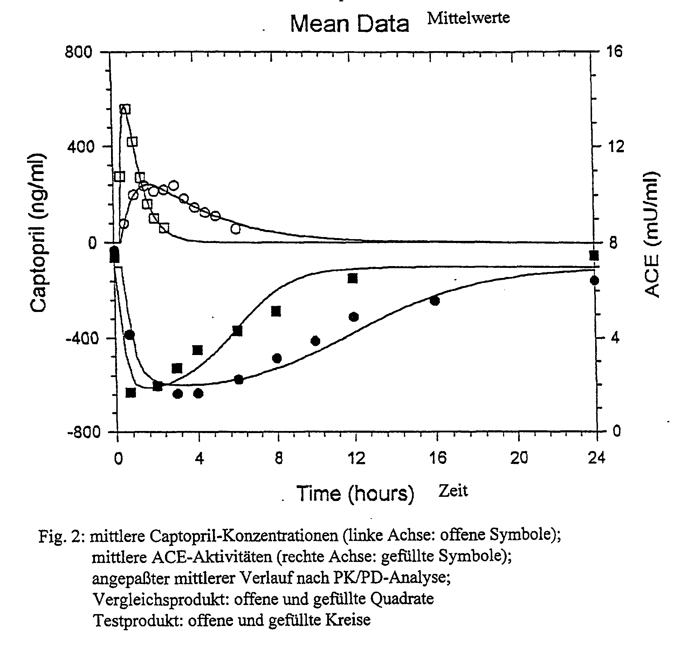

In einer offenen cross-over Studie auf Basis von Einzeldosen wurden Plasmaspiegel und ACE-Hemmung bestimmt. Die Probanden erhielten entweder eine erfindungsgemäße Kapsel mit 150 mg Captopril oder ein Referenzprodukt mit 50 mg Captopril. **Figur 1** zeigt den retardierenden Effekt der Captopril-Kapsel gemäß der Erfindung im Vergleich zu einer schnell freisetzenden Captopril-Tablette. **Figur 2** zeigt Captopril-Plasmaspiegel im Bezug zur ACE-Inhibierung.

### Beispiel 2

### Herstellung:

Für Captopril-Retardkapseln wurden die folgenden drei Komponenten vorgesehen.

| Komponente 1 (Initialdosis): | |
|---|---|
| Captopril (Pulver) | 20 mg |

| Komponente 2 (erste verzögert freisetzende Pelletart): | |
|---|---|
| Captopril | 50 mg |
| Mikrokristalline Cellulose | 49,37 mg |
| Opadry, weiß, bestehend aus Lactose H₂O | 2,07 mg |
| Hydroxypropylmethylcellulose | 1,61 mg |
| Titandioxid | 1,49 mg |
| Macrogol 4000 | 0,58 mg |
| Endragit S 100 | 6,13 mg |
| Dibutylphthalat | 0,61 mg |

| Komponente 3 (zweite verzögert freisetzende Pelletart) | |
|---|---|
| Captopril | 80 mg |
| Mikrokristalline Cellulose | 81,64 mg |
| Opadry, weiß, bestehend aus Lactose H₂O | 3,37 mg |
| Hydroxypropylmethylcellulose | 2,62 mg |
| Titandioxid | 2,43 mg |
| Macrogol 4000 | 0,93 mg |
| Endragit S 100 | 50 mg |
| Dibutylphthalat | 5 mg |

Die Wirkstoffkonzentration von Captopril pro Kapsel beträgt 150 mg.

### Beispiel 3

Für Captopril-Retardkapseln wurden die folgenden drei Komponenten vorgesehen:

| Komponente 1 (Initialdosis): 50 % Captopril | |
|---|---|
| Captopril | 20 mg |
| Mikrokristalline Cellulose | 20,12 mg |

| Komponente 2 (erste verzögert freisetzende Pelletart): 50 % Captopril | |
|---|---|
| Captopril | 50 mg |
| Mikrokristalline Cellulose | 49,36 mg |
| Opadry, weiß, bestehend aus Lactose H₂O | 2,06 mg |
| Hydroxypropylmethylcellulose | 1,61 mg |
| Titandioxid | 1,50 mg |
| Macrogol 4000 | 0,58 mg |
| Endragit S 100 | 6,14 mg |
| Dibutylphthalat | 0,61 mg |

| Komponente 3 (zweite verzögert freisetzende Pelletart): 50 % Captopril | |
|---|---|
| Captopril | 80 mg |
| Mikrokristalline Cellulose | 81,64 mg |
| Opadry, weiß, bestehend aus Lactose H₂O | 3,37 mg |
| Hydroxypropylmethylcellulose | 2,62 mg |
| Titandioxid | 2,43 mg |
| Macrogol 4000 | 0,93 mg |
| Endragit S 100 | 50 mg |
| Dibutylphthalat | 5 mg |

Auch bei diesem Beispiel beträgt die Wirkstoffkonzentration pro Kapsel 150 mg Captopril.

### Beispiel 4:

Zusammensetzung der Captopril-Retardkapseln:

| Komponente 1 (Initialdosis): | |
|---|---|
| Captopril | 20 mg |
| Lactose D80 | 56 mg |
| Mikrokristalline Cellulose | 24 mg |

| Komponente 2 (erste verzögert freisetzende Pelletart): | |
|---|---|
| Captoprilpellets 50 % | 99,93 mg |
| Opadry | 5,78 mg |
| Endragit S 100 | 6,17 mg |
| Dibutylphthalat | 0,62 mg |
| Wasser | 44,33 mg |
| Ethanol 96 % | 34,54 mg |

| Komponente 3 (zweite verzögert freisetzende Pelletart): | |
|---|---|
| Captoprilpellets 50 % | 160,21 mg |
| Opadry | 9,27 mg |
| Endragit S 100 | 49,56 mg |
| Dibutylphthalat | 4,96 mg |
| Wasser | 126,61 mg |
| Ethanol 96 % | 277,56 mg |

Die Gesamtwirkstoffkonzentration einer Retardkapsel beträgt 150 mg Captopril.

## Patentansprüche

1. Pharmazeutische Zubereitung, die folgende Komponenten umfaßt oder aus ihnen besteht:
(i) eine Wirkstoff-Initialdosis, die durch den Wirkstoff neben fakultativen Hilfsstoffen vorgesehen wird,
(ii) eine erste verzögert freisetzende Art Pellets, bei denen der Wirkstoff sowie fakultative Hilfsstoffe von einer Beschichtung umhüllt sind, und
(iii) eine zweite verzögert freisetzende Art Pellets, bei denen der Wirkstoff und fakultative Hilfsstoffe wiederum von einer Beschichtung umhüllt sind,
- wobei der Wirkstoff ein ACE-Hemmer ist,
- wobei die Massen der Beschichtungen gemäß (ii) und (iii) auf Gewichtsbasis im Verhältnisbereich von 1 : 2 bis 1 : 7 vorliegen,
- wobei die Beschichtung der ersten verzögert freisetzenden Art Pellets und die Beschichtung der zweiten verzögert freisetzenden Art Pellets aus demselben magensaftresisten Beschichtungsmaterial bestehen und
- wobei das Verhältnis der Wirkstoffgehalte von Initialdosis: erste verzögert freisetzende Pellets: zweite verzögert freisetzende Pellets im Bereich von 1 : 3 : 3 bis 1 : 10 : 10 liegt.

2. Pharmazeutische Zubereitung, die folgende Komponenten umfaßt oder aus ihnen besteht:
(i) eine Wirkstoff-Initialdosis, die durch den Wirkstoff neben fakultativen Hilfsstoffen vorgesehen wird,
(ii) eine erste verzögert freisetzende Art Pellets, bei denen der Wirkstoff sowie fakultative Hilfsstoffe von einer Beschichtung umhüllt sind, und
(iii) eine zweite verzögert freisetzende Art Pellets, bei denen der Wirkstoff und fakultative Hilfsstoffe wiederum von einer Beschichtung umhüllt sind,
- wobei der Wirkstoff ein ACE-Hemmer ist,
- wobei die Massen der Beschichtungen gemäß (ii) und (iii) auf Gewichtsbasis im Verhältnisbereich von 1 : 2 bis 1 : 7 vorliegen,
- wobei die Beschichtung der ersten verzögert freisetzenden Art Pellets und die Beschichtung der zweiten verzögert freisetzenden Art Pellets aus demselben magensaftresisten Beschichtungsmaterial bestehen und
- wobei das Verhältnis der Wirkstoffgehalte von Initialdosis: erste verzögert freisetzende Pellets: zweite verzögert freisetzende Pellets etwa 1 : 2,5 : 4 beträgt.

3. Zubereitung nach Anspruch 1 oder 2, **dadurch gekennzeichnet, daß** die Massen der Beschichtungen gemäß (ii) und (iii) auf Gewichtsbasis im Verhältnis von etwa 1 : 5 vorliegen.

4. Zubereitung nach Anspruch 1, 2 und/oder 3, **gekennzeichnet durch** Captopril, Moexipril, Perindopril, Quinapril, Ramipril, Spirapril, Tandolapril, deren Gemische und/oder pharmazeutisch unbedenkliche Salze, insbesondere Hydrochloride, insbesondere Perindoprilerbumin, als ACE-Hemmer (angiotensin converting enzyme).

5. Zubereitung nach einem der vorhergehenden Ansprüche, **dadurch gekennzeichnet, daß** bei der Initialdosis der Wirkstoff in Form von Pulver, Granulat und/oder Pellets vorliegt.

6. Zubereitung nach einem der vorhergehenden Ansprüche, **dadurch gekennzeichnet, daß** die Pellets der ersten verzögert freisetzenden Art und/oder die Pellets der zweiten verzögert freisetzenden Art dadurch erhalten worden sind, daß die Initialdosis in Form von Pellets mit der jeweiligen Beschichtung versehen worden ist.

7. Zubereitung nach einem der vorhergehenden Ansprüche, **dadurch gekennzeichnet, daß** es sich bei der Beschichtung der ersten und der zweiten verzögert freisetzenden Pelletart um eine Beschichtung auf Basis von Polymethacrylsäure handelt, insbesondere Eudragit S.

8. Zubereitung nach Anspruch 7, **dadurch gekennzeichnet, daß** die Beschichtung außer Polymethacrylsäure keine weitere Komponente gleicher oder größerer Acidität umfaßt.

9. Zubereitung nach einem der vorhergehenden Ansprüche, **dadurch gekennzeichnet, daß** die Beschichtung übliche Filmbildner und/oder Hilfsstoffe umfaßt, vorzugsweise Dibutylphthalat, Polyethylenglykol, Triethylcitrat (Citroflex), Ethylcellulose (Aquacoat), Titandioxid und/oder Hydroxypropylmethylcellulose.

10. Zubereitung nach einem der vorhergehenden Ansprüche, **gekennzeichnet durch** einen Wirkstoffgehalt der Wirkstoff-Initialdosis von 5 bis 30 Gew.-% des Gesamtwirkstoffgehalts der Zubereitung.

11. Zubereitung nach einem der vorhergehenden Ansprüche, **gekennzeichnet durch** ein Verhältnis (Gewichtsbasis) von erster zu zweiter zu dritter Komponente im Bereich von 1 : 1 : 1 bis 1 : 10 : 10 und insbesondere **durch** ein Verhältnis (Gewichtsbasis) von etwa 1 : etwa 1 : etwa 2.

12. Zubereitung nach einem der vorhergehenden Ansprüche, **dadurch gekennzeichnet, daß** die Initialdosis (insbesondere in Form von Pellets), erste verzögert freisetzende Pellets und/oder zweite verzögert freisetzende Pellets 10- bis 50-proz. im Wirkstoffgehalt sind (Gewichtsbasis), wobei der Wirkstoffgehalt der drei Komponenten gleich oder verschieden sein kann.

13. Zubereitung nach einem der vorhergehenden Ansprüche, **gekennzeichnet durch**
10 bis 30 Gew.-% Initialdosis,
20 bis 40 Gew.-% erste verzögert freisetzende Pelletart und
40 bis 60 Gew.-% zweite verzögert freisetzende Pelletart,
wobei alle drei Komponenten insgesamt 100 Gew.-% ausmachen.

14. Zubereitung nach einem der vorhergehenden Ansprüche, **gekennzeichnet durch**
etwa 22,9 Gew.-% Initialdosis,
etwa 25,8 Gew.-% erste verzögert freisetzende Pelletart und
etwa 51,3 Gew.-% zweite verzögert freisetzende Pelletart,
wobei alle drei Komponenten insgesamt 100 Gew.-% ausmachen.

15. Zubereitung nach einem der vorhergehenden Ansprüche in Form einer Kapsel, insbesondere einer Gelantinekapsel, wobei die Kapsel alle drei Komponenten umfaßt..

16. Zubereitung nach Anspruch 15, **dadurch gekennzeichnet, daß** die Kapsel eine für eine Tagesdosis oder für eine Einmaldosis erforderliche Wirkstoffmenge umfaßt.

17. Zubereitung nach Anspruch 15 oder 16, **dadurch gekennzeichnet, daß** die Kapsel eine für die Tagesdosis oder Einmaldosis erforderliche Captoprilmenge umfaßt, insbesondere im Bereich von 25 bis 300 mg, vorzugsweise 50 bis 200 mg und speziell 75 bis 150 mg.

18. Zubereitung nach einem der vorhergehenden Ansprüche, **dadurch gekennzeichnet, daß** auf Basis einer Tagesdosis oder Einmaldosis bei Captopril als Wirkstoff die Initialdosis 5 bis 30 mg beträgt.

19. Zubereitung nach einem der vorhergehenden Ansprüche, **gekennzeichnet durch** mikrokristalline Zellulose und/oder Lactose als Hilfsstoffe.

## Claims

1. Pharmaceutical preparation which comprises or consists of the following components:
(i) an initial dose of active ingredient, which is provided by the active ingredient together with optional excipients,
(ii) a first delayed-release type of pellet, in which the active ingredient and optional excipients are covered with a coating, and
(iii) a second delayed-release type of pellet, in which the active ingredient and optional excipients are again covered with a coating,
- wherein the active ingredient is an ACE inhibitor,
- wherein the amounts of the coatings according to (ii) and (iii) are present in a ratio, based on weight, within the range of from 1:2 to 1:7.
- wherein the coating of the first delayed-release type of pellet and the coating of the second delayed-release type of pellet consist of the same coating material resistant to gastric juices and
- wherein the ratio of the contents of the active ingredient of initial dose: first delayed-release pellets: second delayed-release pellets is in the range of from 1:3:3 to 1:10:10.

2. Pharmaceutical preparation which comprises or consists of the following components:
(i) an initial dose of active ingredient, which is provided by the active_ingredient together with optional excipients,
(ii) a first delayed-release type of pellet, in which the active ingredient and optional excipients are covered with a coating, and
(iii) a second delayed-release type of pellet, in which the active ingredient and optional excipients are again covered with a coating,
- wherein the active ingredient is an ACE inhibitor,
- wherein the amounts of the coatings according to (ii) and (iii) are present in a ratio, based on weight, within the range of from 1:2 to 1:7.
- wherein the coating of the first delayed-release type of pellet and the coating of the second delayed-release type of pellet consist of the same coating material resistant to gastric juices and
- wherein the ratio of the contents of the active ingredient of initial dose: first delayed-release pellets: second delayed-release pellets is about 1:2.5:4.

3. Preparation according to claim 1 or 2, **characterized in that** the amounts of the coatings according to (ii) and (iii) are present in a ratio, based on weight, of approximately 1:5.

4. Preparation according to claim 1, 2 and/or 3, **characterized by** captopril, moexipril, perindopril, quinapril, ramipril, spirapril, tandolapril, mixtures thereof and/or their pharmaceutically acceptable salts, especially hydrochlorides, especially perindopril erbumin, as ACE (angiotensin converting enzyme) inhibitor.

5. Preparation according to any one of the preceding claims, **characterized in that** the active ingredient in the initial dose is in the form of a powder, granules and/or pellets.

6. Preparation according to any one of the preceding claims, **characterized in that** the pellets of the first delayed-release type and/or the pellets of the second delayed-release type have been obtained by providing the initial dose in the form of pellets with the respective coating.

7. Preparation according to any one of the preceding claims, **characterized in that** the coating for the first and second types of delayed-release pellet is a coating that is based on polymethacrylic acid, more especially Eudragit S.

8. Preparation according to claim 7, **characterized in that** the coating comprises, apart from polymethacrylic acid, no other component of equal or greater acidity.

9. Preparation according to any one of the preceding claims, **characterized in that** the coating comprises customary film-forming agents and/or excipients, especially dibutyl phthalate, polyethylene glycol, triethyl citrate (Citroflex), ethyl cellulose (Aquacoat), titanium dioxide and/or hydroxypropylmethyl cellulose.

10. Preparation according to any one of the preceding claims, **characterized in that** the active ingredient content of the initial dose of active ingredient is from 5 to 30 % by weight of the total active ingredient content of the preparation.

11. Preparation according to any one of the preceding claims, **characterized by** a ratio (based on weight) of first component to second component to third component in the range of from 1:1:1 to 1:10:10, and especially by a ratio (based on weight) of approximately 1 : approximately 1 : approximately 2.

12. Preparation according to any one of the preceding claims, **characterized in that** the active ingredient content of the initial dose (especially in the form of pellets); of the first delayed-release pellets and/or of the second delayed-release pellets is from 10 to 50 % (based on weight), it being possible for the active ingredient contents of the three components to be the same or different.

13. Preparation according to any one of the preceding claims, **characterized by**
initial dose: from 10 to 30 % by weight,
first delayed-release type of pellet: from 20 to 40 % by weight, and
second delayed-release type of pellet: from 40 to 60 % by weight,
the sum of all three components being 100 % by weight.

14. Preparation according to any one of the preceding claims, **characterized by**
initial dose: approximately 22.9 % by weight,
first delayed-release type of pellet: approximately 25.8 % by weight, and
second delayed-release type of pellet: approximately 51.3 % by weight,
the sum of all three components being 100 % by weight.

15. Preparation according to any one of the preceding claims in the form of a capsule, especially a gelatin capsule, the capsule comprising all three components.

16. Preparation according to claim 15, **characterized in that** the capsule comprises an amount of active ingredient required for a daily dose or for a single dose.

17. Preparation according to claim 15 or 16, **characterized in that** the capsule comprises an amount of captopril required for the daily dose or single dose, especially in the range of from 25 to 300 mg, more especially from 50 to 200 mg and very especially from 75 to 150 mg.

18. Preparation according to any one of the preceding claims, **characterized in that**, based on a daily dose or a single dose, with captopril as active ingredient, the initial dose is from 5 to 30 mg.

19. Preparation according to any one of the preceding claims, **characterized by** microcrystalline cellulose and/or lactose as excipients.

## Revendications

1. Composition pharmaceutique, contenant les composants suivants ou constituée des composants suivants :
(i) une dose initiale de principe actif, sous forme de principe actif en présence d'adjuvants facultatifs,
(ii) un premier type de pellets à libération retardée, dans lesquels le principe actif et des adjuvants facultatifs sont enrobés d'un revêtement,
(iii) un deuxième type de pellets à libération retardée, dans lesquels le principe actif et des adjuvants facultatifs sont également enrobés d'un revêtement,
le principe actif étant un inhibiteur de l'ACE,
le rapport en poids du revêtement des pellets (ii) au revêtement des pellets (iii) étant de 1 : 2 à 1 : 7,
les revêtements des pellets à libération retardée du premier et deuxième type étant constitués d'un même matériau d'enrobage résistant au suc gastrique, et
le rapport teneur en principe actif dans la dose initiale : teneur en principe actif dans les pellets du premier type : teneur en principe actif dans les pellets du deuxième type étant de 1:3:3 à 1:10:10.

2. Composition pharmaceutique contenant les composants suivants ou constituée des composants suivants :
(i) une dose initiale de principe actif, sous forme de principe actif en présence d'adjuvants facultatifs,
(ii) un premier type de pellets à libération retardée, dans lesquels le principe actif et des adjuvants facultatifs sont enrobés d'un revêtement, et
(iii) un deuxième type de pellets à libération retardée, dans lesquels le principe actif et des adjuvants facultatifs sont également enrobés d'un revêtement,
le principe actif étant un inhibiteur de l'ACE,
le rapport en poids du revêtement des pellets (ii) au revêtement des pellets (iii) étant de 1:2 à 1:7,
les revêtements des pellets à libération retardée du premier et deuxième type étant constitué d'un même matériau d'enrobage résistant au suc gastrique, et
le rapport teneur en principe actif dans la dose initiale : teneur en principe actif dans les pellets du premier type : teneur en principe actif dans les pellets du deuxième type étant d'environ 1:2,5:4.

3. Composition selon la revendication 1 ou 2, **caractérisée par le fait que** le rapport en poids du revêtement des pellets (ii) au revêtement des pellets (iii) est égal à environ 1:5.

4. Composition selon la revendication 1, 2 et/ou 3, **caractérisée par le fait que** l'inhibiteur de l'ACE (*angiotensin converting enzyme*) est le captopril, le moexipril, le périndopril, le quinapril, le ramipril, le spirapril, le tandolapril, un mélange de ces principes actifs et/ou un sel pharmacologiquement acceptable de ces principes actifs, en particulier un chlorhydrate, et est en particulier le périndoprilerbumine.

5. Composition selon l'une des revendications précédentes, **caractérisée par le fait que**, dans la dose initiale, le principe actif est présent sous forme de poudre, de granulés et/ou de pellets.

6. Composition selon l'une des revendications précédentes, **caractérisée par le fait que** les pellets à libération retardée du premier type et/ou les pellets à libération retardée du deuxième type ont été préparés par enrobage de la dose initiale sous forme de pellets avec le revêtement correspondant.

7. Composition selon une des revendications précédentes, **caractérisée par le fait que** le revêtement des pellets à libération retardée du premier type et du deuxième type est à base de poly(acide méthacrylique), en particulier à base de Eudragit S.

8. Composition selon la revendication 7, **caractérisé par le fait que** le revêtement ne contient pas d'autres composants ayant une acidité équivalente à celle du poly(acide méthacrylique) ou plus forte que celle-ci.

9. Composition selon l'une des revendication précédentes, **caractérisée par le fait que** le revêtement contient des agents filmogènes et/ou des adjuvants usuels, de préférence du phtalate de dibutyle, du polyéthylèneglycol, du citrate de triéthyle (Citroflex), de l'éthylcellulose (Aquacoat), du dioxyde de titane et/ou de l'hydroxypropylméthylcellulose.

10. Composition selon l'une des revendications précédentes, **caractérisée par le fait que** la quantité de principe actif dans la dose initiale représente de 5 à 30 % en poids de la quantité totale de principe actif dans la composition.

11. Composition selon l'une des revendications précédentes, **caractérisée par le fait que** le rapport en poids premier composant : deuxième composant : troisième composant est de 1 : 1 : 1 à 1 : 10 : 10 et vaut en particulier environ 1 : environ 1 : environ 2.

12. Composition selon l'une quelconque des revendications précédentes, **caractérisée par le fait que** la dose initiale (en particulier sous forme de pellets) et les pellets à libération retardée du premier et/ou du deuxième type ont une teneur en principe actif comprise de 10 à 50 % en poids, les teneurs en principe actif de ces trois composants pouvant être identiques ou différentes.

13. Composition selon l'une des revendications précédentes, **caractérisée par le fait qu'**elle contient
de 10 à 30 % en poids de dose initiale,
de 20 à 40 % en poids de pellets à libération retardée du premier type, et
de 40 à 60 % en poids de pellets à libération retardée du deuxième type,
l'ensemble de ces composants se complétant à 100 % en poids.

14. Composition selon l'une des revendications précédentes, **caractérisée par le fait qu'**elle contient
environ 22,9 % en poids de dose initiale,
environ 25,8 % en poids de pellets à libération retardée du premier type,
et environ 51,3 % en poids de pellets à libération retardée du deuxième type,
l'ensemble de ces composants se complétant à 100 % en poids.

15. Composition selon l'une des revendications précédentes sous forme d'une gélule, en particulier sous forme d'une gélule en gélatine, laquelle gélule renferme l'ensemble des trois composants.

16. Composition selon la revendication 15, **caractérisée par le fait que** la gélule comprend la quantité de principe actif pour une dose quotidienne ou pour une dose unique.

17. Composition selon la revendication 15 ou 16, **caractérisée par le fait que** la gélule comprend la quantité de caprotil pour une dose quotidienne ou pour une dose unique, en particulier une quantité de 25 à 300 mg, de préférence de 50 à 200 mg et en particulier de 75 à 150 mg.

18. Composition selon l'une des revendications précédentes, **caractérisée par le fait que**, sur la base d'une dose quotidienne ou d'une dose unique de captopril en tant que principe actif, la dose initiale est de 5 à 30 mg.

19. Composition selon l'une des revendications précédentes, **caractérisée par le fait qu'**elle contient, en tant qu'adjuvants, de la cellulose microcristalline et/ou du lactose.
